# EUROPÄISCHE PATENTANMELDUNG

(11) **EP 2 181 723 A2**
(43) Veröffentlichungstag der Anmeldung: **05.05.2010**
(21) Anmeldenummer: 09170258.9
(22) Anmeldetag: 15.09.2009
(51) Int. Cl.: A61L 31/14, A61L 31/08, A61L 31/02

(54) **Implantat aus einer biokorrodierbaren Eisen- oder Magnesiumlegierung**

(30) Priorität: 29.10.2008 DE 102008043277
(71) Anmelder: Biotronik VI Patent AG, 6341 Baar (CH)
(72) Erfinder: Wittchow, Eric, 90403, Nürnberg (DE)
(74) Vertreter: Lindner-Vogt, Karin L.

(57) **Zusammenfassung**

Die Erfindung betrifft ein Implantat aus einem biokorrodierbaren metallischen Werkstoff und mit einer Beschichtung bestehend aus oder enthaltend ein biokorrodierbares Polyphosphat, Polyphosphonat oder Polyphosphit.

## Beschreibung

### Technisches Gebiet

Die Erfindung betrifft ein Implantat aus einer biokorrodierbaren Eisen- oder Magnesiumlegierung, das eine polymere Beschichtung aufweist sowie eine neue Verwendung eines Polyphosphoesters.

### Hintergrund der Erfindung und Stand der Technik

Implantate haben in vielfältiger Ausführungsform Anwendung in der modernen Medizintechnik gefunden. Sie dienen beispielsweise der Unterstützung von Gefäßen, Hohlorganen und Gangsystemen (Endovaskuläre Implantate), zur Befestigung und temporären Fixierung von Gewebeimplantaten und Gewebstransplantationen, aber auch zu orthopädischen Zwecken, zum Beispiel als Nagel, Platte oder Schraube.

So hat sich zum Beispiel die Implantation von Stents als eine der wirkungsvollsten therapeutischen Maßnahmen bei der Behandlung von Gefäßerkrankungen etabliert. Stents haben den Zweck, in Hohlorganen eines Patienten eine Stützfunktion zu übernehmen. Stents herkömmlicher Bauart weisen dazu eine filigrane Tragstruktur aus metallischen Streben auf, die zur Einbringung in den Körper zunächst in einer komprimierten Form vorliegt und am Ort der Applikation aufgeweitet wird. Einer der Hauptanwendungsbereiche solcher Stents ist das dauerhafte oder temporäre Weiten und Offenhalten von Gefäßverengungen, insbesondere von Verengungen (Stenosen) der Herzkranzgefäße. Daneben sind beispielsweise auch Aneurysmenstents bekannt, die zur Stützung beschädigter Gefäßwände dienen.

Stents besitzen eine Umfangswandung von ausreichender Tragkraft, um das verengte Gefäß im gewünschten Maße offen zu halten, und einen rohrförmigen Grundkörper durch den der Blutfluss ungehindert weiterläuft. Die tragende Umfangswandung wird in der Regel von einer gitterartigen Tragstruktur gebildet, die es erlaubt, den Stent in einem komprimierten Zustand mit kleinem Außendurchmesser bis zur behandelnden Engstelle des jeweiligen Gefäßes einzuführen und dort beispielsweise mit Hilfe eines Ballonkatheters soweit aufzuweiten, dass das Gefäß den gewünschten, vergrößerten Innendurchmesser aufweist. Um unnötige Gefäßbeschädigungen zu vermeiden, sollte der Stent nach dem Aufweiten und nach Entfernen des Ballons nicht oder nur in geringem Umfang elastisch zurückfedern, so dass der Stent beim Aufweiten nur wenig über den gewünschten Enddurchmesser hinaus geweitet werden muss. Weitere Kriterien, die in Bezug auf einen Stent wünschenswert sind, umfassen beispielsweise eine gleichmäßige Flächenabdeckung und eine Struktur, die eine gewisse Flexibilität in Bezug auf die Längsachse des Stents erlaubt. In der Praxis wird zur Realisation der genannten mechanischen Eigenschaften der Stent in der Regel aus einem metallischen Werkstoff geformt.

Neben den mechanischen Eigenschaften eines Stents sollte dieser aus einem biokompatiblen Material bestehen, um Abstoßungsreaktionen vorzubeugen. Derzeit werden bei etwa 70% aller perkutanen Interventionen Stents eingesetzt, in 25% aller Fälle kommt es jedoch zu einer In-Stent Restenose aufgrund eines überschießenden neointimalen Wachstums, das durch eine starke Proliferation der arteriellen glatten Muskelzellen und eine chronische Entzündungsreaktion hervorgerufen wird. Zur Senkung der Restenoseraten werden verschiedene Lösungsansätze verfolgt.

Ein Ansatz zur Minderung der Restenoserate sieht vor, auf dem Stent eine pharmazeutisch aktive Substanz (Wirkstoff) bereitzustellen, die den Mechanismen der Restenose entgegenwirkt und den Heilungsverlauf unterstützt. Der Wirkstoff wird in Reinform oder eingebettet in eine Trägermatrix als Beschichtung aufgetragen oder in Kavitäten des Implantats gefüllt. Beispiele umfassen die Wirkstoffe Sirolimus und Paclitaxel.

Ein weiterer, aussichtsreicher Ansatz zur Lösung des Problems liegt in der Verwendung biokorrodierbarer Metalle und deren Legierungen, denn zumeist ist eine dauerhafte Stützfunktion durch den Stent nicht erforderlich; das zunächst geschädigte Körpergewebe regeneriert. So wird beispielsweise in DE 197 31 021 A1 vorgeschlagen, medizinische Implantate aus einem metallischen Werkstoff zu formen, dessen Hauptbestandteil Eisen, Zink oder Aluminium sind bzw. ein Element aus der Gruppe der Alkalimetalle oder Erdalkalimetalle. Als besonders geeignet werden Legierungen auf Basis von Magnesium, Eisen und Zink beschrieben. Nebenbestandteile der Legierungen können Mangan, Kobalt, Nickel, Chrom, Kupfer, Cadmium, Blei, Zinn, Thorium, Zirkonium, Silber, Gold, Palladium, Platin, Silizium, Calcium, Lithium, Aluminium, Zink und Eisen sein. Weiterhin ist aus der DE 102 53 634 A1 der Einsatz einer biokorrodierbaren Magnesiumlegierung mit einem Anteil von Magnesium >90%, Yttrium 3,7 - 5,5%, Seltenerdmetallen 1,5 - 4,4% und Rest < 1% bekannt, die sich insbesondere zur Herstellung einer Endoprothese, z. B. in Form eines selbstexpandierenden oder ballonexpandierbaren Stents, eignet. Der Einsatz von biokorrodierbaren metallischen Werkstoffen in Implantaten dürfte zu einer deutlichen Minderung von Abstoßungs- oder Entzündungsreaktionen führen.

Die häufig sauren Produkte des Abbaus bekannter biokorrodierbarer Polymere können zu einer inflammatorischen Reaktion des umgebenden Gewebes führen, d.h. das Material zeigt nur eine mäßige Biokompatibilität. So wurde zum Beispiel nachgewiesen, dass bei biodegradierbaren Polyorthoestern weder das Polymer selber, noch die Zwischenprodukte während der Degradation für die Inflammation verantwortlich sind, sondern die freiwerdende Essigsäure (Zignani et al., Subconjunctival biocompatibility of a viscous bioerodible poly(ortho ester), J. Biomed. Mater. Res., 1997, 39 p. 277 - 285). Daneben sind noch andere, vor allem prozesstechnisch bedingte Ursachen für eine schlechte Biokompatibilität bekannt.

Neben der unerwünschten biologischen Antwort auf die sauren Degradationsprodukte in Form einer Inflammation beeinflusst der geänderte pH-Wert im Falle eines Implantats aus einer biokorrodierbaren Magnesiumlegierung auch die Ausbildung einer Passivierungsschicht, die üblicherweise die Degradation des Implantate nach Kontakt mit Feuchtigkeit oder Blut deutlich verlangsamt. Wird der pH-Wert an der Passivierungsschicht durch Freisetzung sauer Abbauprodukte gesenkt, so wird die Ausbildung der hyrdoxidhaltigen Passivierungsschicht beeinträchtigt und so die Degradation in der Regel beschleunigt. Dadurch verliert zum Beispiel ein Stent aus einer biokorrodierbaren Magnesiumlegierung schneller seine Stützkraft. Die genannten negativen Effekte wurden in zahlreichen eigenen Versuchen beobachtet, in denen die Kombination von Polymeren mit saueren Abbauprodukten, wie Polyestern (PLA, PLGA oder P4BH), Polyanhydriden oder Polyesteramiden mit einem Stent aus einer biokorrodierbaren Magnesiumlegierung untersucht wurden. Allgemein ist dies für alle Polymere gültig, deren Kettenaufbau über eine chemische Reaktion einer oder mehrerer Carbonsäurefunktionen der entsprechenden Monomere erfolgt.

### Zusammenfassung der Erfindung

Der Erfindung liegt die Aufgabe zugrunde, ein oder mehrere der geschilderten Probleme zu mindern oder zu überwinden.

Ein erster Aspekt der Erfindung liegt in der Bereitstellung eines Implantats aus einer biokorrodierbaren Eisen- oder Magnesiumlegierung und mit einer Beschichtung bestehend aus oder enthaltend ein biokorrodierbares Polyphosphat, Polyamidophosphat, Polyphosphonat oder Polyphosphit. Der Erfindung liegt die Erkenntnis zu Grunde, dass die Degradation der genannten Polyphosphoester nicht zu saueren Abbauprodukten führt, da der Kettenaufbau nicht über Carbonsäurefunktionen erfolgt. Durch die Verwendung von degradierbaren Polymeren, deren Abbauprodukte nicht sauer, sondern neutral oder sogar leicht basisch reagieren, kann die Ausbildung der Passivierungsschicht an der Oberfläche des Implantats aus einer biokorrodierbaren Metallschicht unterstützt werden.

Polyphosphoester sind Polymere mit einer linearen Grundstruktur aus kovalent gebundenen Monomeren, die eine hydrophile Phosphat-, Amidophosphat-, Phosphonat- oder Phosphitgruppe und eine die phosphorhaltigen Gruppen im Polymer verknüpfende hydrophobe Gruppe beinhalten.

An die Phosphat- oder Phosphonatgruppe kann ferner ein substituierter oder unsubstituierter Alkylrest gebunden sein. Über die hydrophobe Gruppe beziehungsweise den Alkylrest kann die Lipophilie des Polymers und damit die Degradationsgeschwindigkeit beeinflusst werden. Dabei wird in der Regel mit zunehmender Lipophilie des Polymers die Degradationsgeschwindigkeit herabgesetzt. Polyphosphoester, insbesondere Poly(alkylenphosphate), zeigen eine sehr geringe Cytotoxizität (Wan et al., J. Am. Chem. Soc., 2001, 123, pp. 9480 - 9481). Der synthetische Zugang zu Poly(alkylenphosphaten) kann über eine ringöffnende Polymerisation fünf- oder sechsgliedriger, zyklischer Ester der Phosphorsäure und seiner Derivate erfolgen (Penczek et al., Biomacromolecules, 2005, 6, pp. 547 - 551). Die Polymere sind in der Regel in Alkoholen (insbesondere Methanol) löslich und können zum Beispiel über übliche Spray-Methode (evtl. mit einem Wirkstoff gemischt) auf das Implantat aufgetragen werden.

Besonders einfach steuert man die Eigenschaften des Polymers, indem die Hauptkette unverändert bleibt und im letzten Syntheseschritt ein geeigneter Substituent an die Phosphat- oder Phosphonatgruppe gebunden wird. Während die Hauptkette des Polymers zu neutralen Diolen und Phosphat zerfällt, lassen sich durch eine Variation des Substituenten die Eigenschaften des Polymers steuern:
- Eine Aminogruppe am Substituenten bewirkt eine schnellere Degradation des Polymers, vermutlich durch einen autokatalytischen Effekt und eine Erhöhung des pH-Werts durch die bei Abbau freiwerdenden Amine.
- Eine Methyl- oder Ethylgruppe als Substituent verlangsamt die Degradation merklich.

Wird ein Polyphosphit durch Umsetzung mit Chlor aktiviert, kann es in einem zweiten Schritt mit unterschiedlichen nukleophilen Substanzen umgesetzt werden. Dies können z.B. Aminosäuren oder Oligopeptide sein, wodurch Polyamidophosphate erhalten werden.

Der Substituent kann gegebenenfalls auch zur Anbindung eines pharmakologischen Wirkstoffs genutzt werden, der erst beim Abbau des Polymers freigesetzt wird. So kann der Substituent beispielsweise eine Nitrogruppe aufweisen, die unter Freisetzung von NO im Körper metabolisiert und so zu einer lokalen, gewünschten Gefäßdilatation führen. Auch komplexere pharmakologisch wirksame Verbindungen können direkt über das entsprechende Chlorid an ein Polyphosphit gebunden werden, falls sie eine reaktive Aminfunktion oder eine Hydroxygruppe besitzen. Beispiele für zur Anbindung geeignete Wirkstoffe umfassen Amlopidin (Anbindung über NH₂), Bosentan, Paclitaxel und Sirolimus (jeweils Anbindung über OH).

Vorzugsweise ist das biokorrodierbare Polymer ein Poly(alkylenphosphat) der Formel (I) 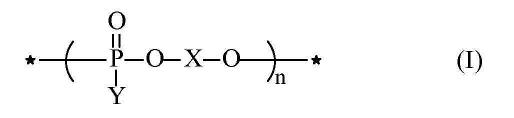 wobei Y steht für
- NR₁R₂ mit R₁ und R₂ unabhängig voneinander gewählt = H oder ein substituierter oder unsubstituierter C1-C10-Alkylrest;
- OR mit R = H oder ein substituierter oder unsubstituierter C1-C10-Alkylrest; oder
- eine Aminosäure, die über ihre Aminfunktion an P gebunden ist; und
X eine substituierte oder unsubstituierte Ethylen- oder Propylenbrücke ist.

Insbesondere ist Y eine Aminosäure ausgewählt aus der Gruppe Lysin, Arginin, Histidin, Alanin und Phenylalanin.

Eine Beschichtung im Sinne der Erfindung ist eine zumindest abschnittsweise Auftragung der Komponenten auf den Grundkörper des Implantats, insbesondere Stents. Vorzugsweise wird die gesamte Oberfläche des Grundkörpers des Implantats/Stents von der Beschichtung bedeckt. Eine Schichtdicke liegt vorzugsweise im Bereich von 1 nm bis 100 µm, besonders bevorzugt 300 nm bis 15 µm. Die Beschichtung besteht aus einem biokorrodierbaren Polyphosphoester oder sie enthält einen solchen Polyphosphoester. Der Gewichtsanteil von Polyphosphoester an den die Trägermatrix bildenden Komponenten der Beschichtung beträgt mindestens 30%, vorzugsweise mindestens 50%, besonders bevorzugt mindestens 70%. Es kann ein Blend aus verschiedenen Polyphosphoestern vorliegen. Die Komponenten der Beschichtung umfassen die als Trägermatrix agierenden Materialien, also Materialien die für die funktionellen Eigenschaften der Trägermatrix notwendig sind, z. B. auch Hilfsstoffe zur Verbesserung der Viskositätseigenschaften, Gelbildung und Verarbeitbarkeit. Diese Komponenten umfassen nicht die gegebenenfalls zugesetzten Wirkstoffe oder Markermaterialien. Die Beschichtung wird direkt auf die Implantatoberfläche aufgebracht oder es wird erst eine Haftvermittler-Schicht eingesetzt. Diese können z.B. auf die Oberfläche des Grundmaterials aufgebrachte Silane oder Phosphonate mit einer reaktiven Endgruppe (COOH, OH, NH₂, Aldehyd) oder eine oxidische Konversionsschicht des Grundmaterials sein.

Die erfindungsgemäß eingesetzten Polyphosphoester sind hochgradig biokompatibel und biokorrodierbar. Die Verarbeitung kann nach Standardverfahren für die Beschichtung erfolgen. Es können einschichtige, aber auch mehrschichtige Systeme (z.B. so genannte base coat, drug coat oder top coat - Schichten) erstellt werden.

Das Polymer kann als Trägermatrix für pharmazeutische Wirkstoffe, Röntgenmarker oder Magnetresonanzmarker agieren. Der Röntgenmarker kann bei Implantaten aus einem biokorrodierbaren metallischen Werkstoff nicht direkt auf das Produkt aufgebracht werden, da er z. B. die Degradation des Stents durch Bildung von Lokalelementen beeinflussen würde. In der Matrix aus Polyphosphoester ist der Marker dagegen vom Grundkörper abgeschirmt.

Unter einer biokorrodierbaren Eisen- oder Magnesiumlegierung im Sinne der Erfindung wird ein metallisches Gefüge verstanden, dessen Hauptkomponente Eisen oder Magnesium ist. Hauptkomponente ist die Legierungskomponente, deren Gewichtsanteil an der Legierung am höchsten ist. Ein Anteil der Hauptkomponente beträgt vorzugsweise mehr als 50 Gew.%, insbesondere mehr als 70 Gew.%. Vorzugsweise enthält die biokorrodierbare Magnesiumlegierung Yttrium und weitere Seltenerdmetalle, da sich eine derartige Legierung aufgrund ihrer physiko-chemischen Eigenschaften und hohen Biokompatibilität, insbesondere auch seiner Abbauprodukte, auszeichnet. Besonders bevorzugt wird eine Magnesiumlegierung der Zusammensetzung Seltenerdmetalle 5,2 - 9,9 Gew.%, davon Yttrium 3,7 - 5,5 Gew.%, und Rest < 1 Gew.%, wobei Magnesium den auf 100 Gew.% fehlenden Anteil an der Legierung einnimmt, eingesetzt. Diese Magnesiumlegierung bestätigte bereits experimentell und in ersten klinischen Versuchen ihre besondere Eignung, d. h. zeigt eine hohe Biokompatibilität, günstige Verarbeitungseigenschaften, gute mechanische Kennwerte und ein für die Einsatzzwecke adäquates Korrosionsverhalten. Unter der Sammelbezeichnung "Seltenerdmetalle" werden vorliegend Scandium (21), Yttrium (39), Lanthan (57) und die 14 auf Lanthan (57) folgenden Elemente, nämlich Cer (58), Praseodym (59), Neodym (60), Promethium (61), Samarium (62), Europium (63), Gadolinium (64), Terbium (65), Dysprosium (66), Holmium (67), Erbium (68), Thulium (69), Ytterbium (70) und Lutetium (71) verstanden.

Der Polyphosphoester und die Eisen- oder Magnesiumlegierung sind so in ihrer Zusammensetzung zu wählen, dass sie biokorrodierbar sind. Als Prüfmedium zur Testung des Korrosionsverhaltens von polymeren Werkstoffen oder Legierungen dient künstliches Plasma, wie es nach EN ISO 10993-15:2000 für Biokorrosionsuntersuchungen vorgeschrieben ist (Zusammensetzung NaCl 6,8 g/l, CaCl₂ 0,2 g/l, KCI 0,4 g/l, MgSO₄ 0,1 g/l, NaHCO₃ 2,2 g/l, Na₂HPO₄ 0,126 g/l, NaH₂PO₄ 0,026 g/I). Eine Probe des zu untersuchenden Werkstoffs wird dazu in einem verschlossenen Probenbehälter mit einer definierten Menge des Prüfmediums bei 37°C gelagert. In zeitlichen Abständen - abgestimmt auf das zu erwartende Korrosionsverhalten - von wenigen Stunden bis zu mehreren Monaten werden die Proben entnommen und in bekannter Weise auf Korrosionsspuren untersucht. Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen.

Implantate im Sinne der Erfindung sind über ein chirurgisches Verfahren in den Körper eingebrachte Vorrichtungen und umfassen Befestigungselemente für Knochen, beispielsweise Schrauben, Platten oder Nägel, chirurgisches Nahtmaterial, Darmklammern, Gefäßclips, Prothesen im Bereich des Hart- und Weichgewebes und Ankerelemente für Elektroden, insbesondere von Schrittmachern oder Defibrillatoren.

Vorzugsweise ist das Implantat ein Stent. Stents herkömmlicher Bauart weisen eine filigrane Stützstruktur aus metallischen Streben auf, die zur Einbringung in den Körper zunächst in einem nicht-expandierten Zustand vorliegt und die am Ort der Applikation dann in einen expandierten Zustand aufgeweitet wird. Aufgrund der Verwendungsweise sind spröde Beschichtungssysteme ungeeignet; Polyphosphoester haben dagegen besonders geeignete Materialeigenschaften, wie eine für die Zwecke hinreichende Viskosität und Flexibilität. Der Stent kann vor oder nach dem Crimpen auf einen Ballon beschichtet werden.

Ein zweiter Aspekt der Erfindung bezieht sich auf die Verwendung von biokorrodierbaren Polyphosphoestern als Beschichtungsmaterial für einen Stent aus einer biokorrodierbaren Eisen- oder Magnesiumlegierung.

Die Erfindung wird nachfolgend anhand eines Ausführungsbeispiels näher erläutert.

### Darstellung von Poly(2-aminobenzyl-propylenphosphat)

Subsituierte Polyphosphonate können über entsprechende Polyphosphite hergestellt werden. Das entsprechende Polyphosphit kann durch eine ringöffnende Polymerisation hergestellt werden, da so größere Molmassen (Mₙ > 10⁵) bei der Herstellung möglich sind, als durch Polykondensation. Die Herstellung erfolgt analog Literaturvorschrift (Penczek et al., Makromol. Chem. 1977, 178, pp. 2943-2947):

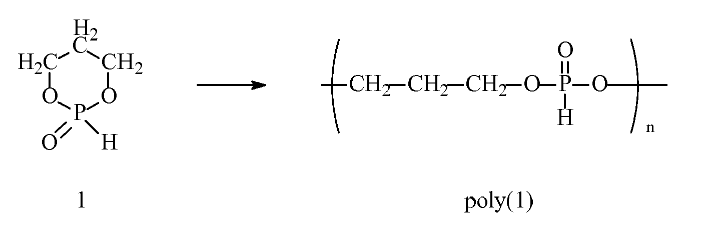

Eine Lösung von 7mol Oxyphosphonoyloxytrimethylen (1) und 3x10-²mol/L [(*i*-C₄H₉)₃Al] wird bei 25°C in 1000ml trockenem THF für 24 Stunden bis zur Gleichgewichtseinstellung umgesetzt. Das Produkt wird in trockenem Toluol ausgefällt. Das Poly(oxyphosphonoyloxytrimethylen) (poly(1)) fällt dabei als weißes, hydrolyseempfindliches Pulver in einer Ausbeute von 50% an.

In eine 10%ige Lösung aus Polymer (poly(1)) in trockenem CH₂Cl₂ wird bei 0°C solange trockenes Cl₂-Gas eingeleitet, bis eine dauerhafte gelbe Färbung entsteht. Überschüssiges Cl₂ wird anschließend wieder im Vakuum entfernt, bis eine klare Lösung von Poly(alkenylchlorophosphat) (2) entsteht (Vorschrift analog Penczek et al. Macromolecules 1993, 26, pp. 2228-2233). Zu der klaren Lösung aus Poly(alkenylchlorophosphat) (2) in CH₂Cl₂ wird bei RT tropfenweise eine Lösung 215mol% Benzylamin in CH₂Cl₂ über 1 Stunde zugetropft. Das Reaktionsgemisch wird eine weitere Stunde bei 0°C gerührt, wobei Benzylaminhydrochlorid ausfällt. Nach Abfiltrieren des Hydrochlorids erhält man eine klare Lösung, die im Vakuum auf 15-20% ihres Ausgangsvolumens aufkonzentriert wird, ehe das Produkt Poly(2-aminobenzyl-propylenphosphat) (3) aus Acetonitril ausgefällt und getrocknet wird (Vorschrift analog Penczek et al. Macromolecules 1986, 19, pp. 2228-2233).

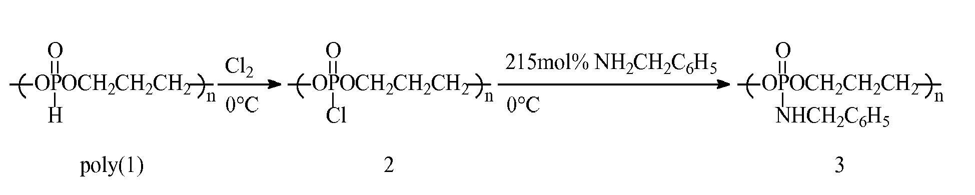

### Ausführungsbeispiel - Beschichtung eines Stents

Ein Stent aus der biokorrodierbaren Magnesiumlegierung WE43 (4 Gew.% Yttrium, 3 Gew.% Seltene Erden außer Yttrium, Rest Magnesium und herstellungsbedingte Verunreinigungen) wird wie folgt beschichtet:

Es wird eine Lösung von Poly(2-aminobenzyl-propylenphosphat) (3) in CH₂Cl₂ angesetzt (30 Gew.%). Der Stent wird von Staub und Rückständen gereinigt und in eine geeignete Stentbeschichtungsapparatur (DES Coater, Eigenentwicklung Fa. Biotronik) eingespannt. Mit Hilfe eines Airbrush Systems (Fa. EFD oder Spraying System) wird der sich drehende Stent unter konstanten Umgebungsbedingungen (Raumtemperatur; 42% Luftfeuchte) halbseitig mit der Lösung beschichtet. Bei einem Düsenabstand von 20 mm ist ein 18 mm langer Stent nach ca. 10 min beschichtet. Nach Erreichen der beabsichtigten Schichtmasse wird der Stent 5 min bei RT getrocknet, ehe nach Drehen des Stents und erneutem Einspannen die unbeschichtete Seite auf dieselbe Weise beschichtet wird. Der fertig beschichtete Stent wird für 36 h bei 40°C in einem Vakuumofen (Vakucell; Fa. MMM) getrocknet.

Eine Schichtdicke des aufgetragenen Polyphosphoesters beträgt etwa 2 bis 7 µm.

## Patentansprüche

1. Implantat aus einer biokorrodierbaren Eisen- oder Magnesiumlegierung und mit einer Beschichtung bestehend aus oder enthaltend ein biokorrodierbares Polyphosphat, Polyphosphonat oder Polyphosphit.

2. Implantat nach Anspruch 1, bei dem das Implantat ein Stent ist.

3. Implantat nach Anspruch 1 oder 2, bei dem die Beschichtung einen Wirkstoff enthält.

4. Implantat nach einem der vorhergehenden Ansprüche, mit einem Poly(alkylenphosphat) der Formel (I) 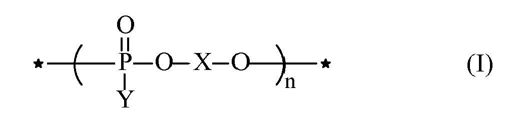 wobei Y steht für
- NR₁R₂ mit R₁ und R₂ unabhängig voneinander gewählt = H oder ein substituierter oder unsubstituierter C1-C10-Alkylrest;
- OR mit R = H oder ein substituierter oder unsubstituierter C1-C10-Alkylrest; oder
- eine Aminosäure, die über ihre Aminfunktion an P gebunden ist; und
X eine substituierte oder unsubstituierte Ethylen- oder Propylenbrücke ist.

5. Verwendung eines biokorrodierbaren Polyphosphats, Polyphosphonats oder Polyphosphits als Beschichtungsmaterial für einen Stent aus einem biokorrodierbaren metallischen Werkstoff.
